# NEW EUROPEAN PATENT SPECIFICATION

(11) **EP 1 011 570 B2**
(45) Date of publication and mention of the opposition decision: **01.06.2011**
(45) Mention of the grant of the patent: 09.05.2007
(21) Application number: 97948486.2
(22) Date of filing: 21.11.1997
(51) Int. Cl.: A61F 13/15

(54) **PROCESS FOR MAKING A LAMINATE COMPRISING PARTICLES**
VERFAHREN ZUM HERSTELLEN EINES TEILCHEN ENTHALTENDEN LAMINATS
PROCEDE DE PRODUCTION D'UN STRATIFIE COMPRENANT DES PARTICULES

(30) Priority: 04.12.1996 EP 96119426
(43) Date of publication of application: 28.06.2000
(73) Proprietor: THE PROCTER & GAMBLE COMPANY, Cincinnati, OH 45202 (US)
(72) Inventor: CARLUCCI, Giovanni, I-66100 Chieti (IT); GAGLIARDI, Ivan, I-65123 Pescara (IT)
(74) Representative: Veronese, Pancrazio
(86) International application number: PCT/US1997/021454
(87) International publication number: WO 1998/024626

(56) References cited:
- EP-A- 0 598 413
- EP-A- 0 682 927
- EP-A- 0 682 927
- WO-A-94/01069
- WO-A-95/03019
- WO-A-95/17868
- WO-A-97/05841
- US-A- 4 715 918
- US-A- 4 752 349
- US-A- 5 059 277
- US-A- 5 415 717

## Description

### Field of the invention

The present invention relates to a process for making a laminate material which is particularly useful in disposable absorbent articles. In particular the process can be used to provide a laminate of liquid impervious film and a liquid pervious nonwoven substrate with a particulate material between them. The film and the nonwoven substrate are attached to each other by thermal bonding for example by spot welding. The process is adapted such that it can be used to produce the laminate as part of a larger process to make absorbent articles.

### Background of the invention

In EP-A-682 927 a shield against particle loss in absorbent products is disclosed which is formed for example by attaching a nonwoven material along an endless line to a film layer, which film layer is used as the backsheet in the absorbent product according to this disclosure. The intend of this disclosure is the isolation of the particles such that they cannot be lost during production, shipment, or use of the product.

Based on this state of the art the problem underlying the present invention is to provide a simple and cost effective process to make a laminate having a continuous bond in longitudinal direction and in which particles are enclosed, but not necessarily isolated.

Similar laminates are also already known, however, they are provided by adhesively joining two layers of continuos non-woven material along their longitudinal edges. In the process according to WO 9503019 the additional requirement of creating a particle free longitudinal window is disclosed since the adhesive cannot join the laminate layer in regions of particle presence.

In respect to this state of the art the problem underlying the present invention is to provide a more robust process in respect to the presence of particles, but also to replace the adhesive (since this causes problems when cutting the laminate in transverse direction through the adhesive). Further the present invention is only directed to a process for making a laminate which has liquid impermeability on one side of the laminate.

Further objectives of the present invention can be letter understood from the following description and drawings.

### Brief description of the invention

The present invention relates to a process for making a laminate which comprises a particulate material. The process is according to claim 1.

Preferably, both continuous webs and the layer of particulate material are aligned along their individual respective longitudinal centrelines to which the individual webs and the layer of particulate material are also symmetrical. The width Wp of the layer of particulate material comprises all particles.

In a preferred embodiment according to the present process the continuous web of liquid pervious substrate and the layer of particulate material have non-linear longitudinal side edges and the thermal bonding follows the non linearity of the side edge contour. In this embodiment the liquid impervious film typically has linear longitudinal side edges.

Preferably, the particulate material comprises odour control materials which are preferably selected from Zeolite, Carbon Black and a ph-buffermaterial. The liquid pervious substrate is preferably fibrous and most preferably comprises fibres which are capable of thermal bonding to the liquid impervious film material, preferably the liquid pervious substrate consists of such fibres. The width Wp of the layer of particulate material comprises all particles.

According to a particular embodiment of the present invention the thermal bonding is provided by a pattern of spot welding sites.

In a particularly preferred embodiment of the process according to the present invention the width Wf of the film layer is larger than the width WI of the liquid pervious layer.

This process can also be used as a part of a larger process for making absorbent articles which articles comprise a backsheet laminate, a liquid pervious topsheet laminate to be worn adjacent the wearer's skin and an absorbent core laminate deposited between the backsheet laminate and the topsheet laminate.

In this larger process for making disposable absorbent articles the backsheet laminate and the core laminate are provided at least partially by the particulate material comprising laminate in which Wf is larger than WI such that the film material is part of the backsheet laminate, preferably the backsheet is provided by the film. Then the particulate material layer and the web of liquid pervious substrate both form part of the core laminate.

### Brief description of the drawings

Figure 1 is a schematic view of the process according to the present invention.
Figure 2 is a perspective, partially cut away view of a laminate made according to one embodiment of the present invention.

### Detailed description of the invention

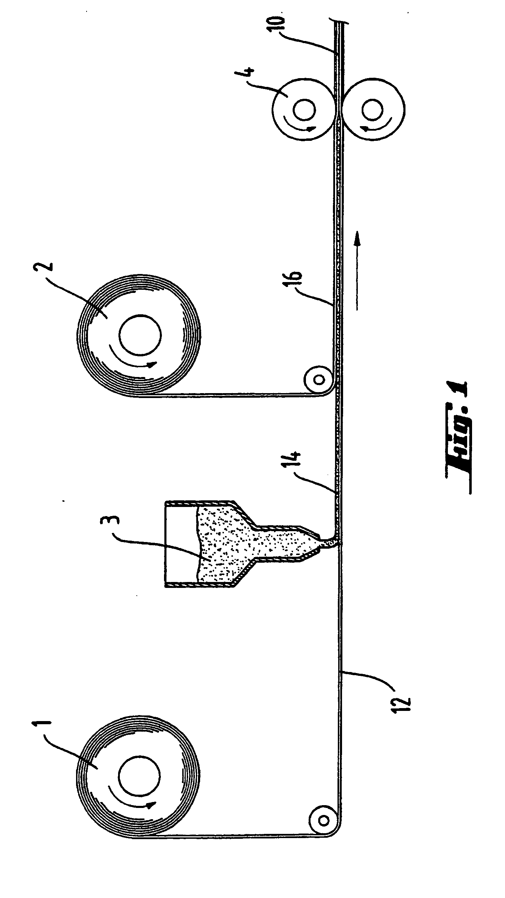

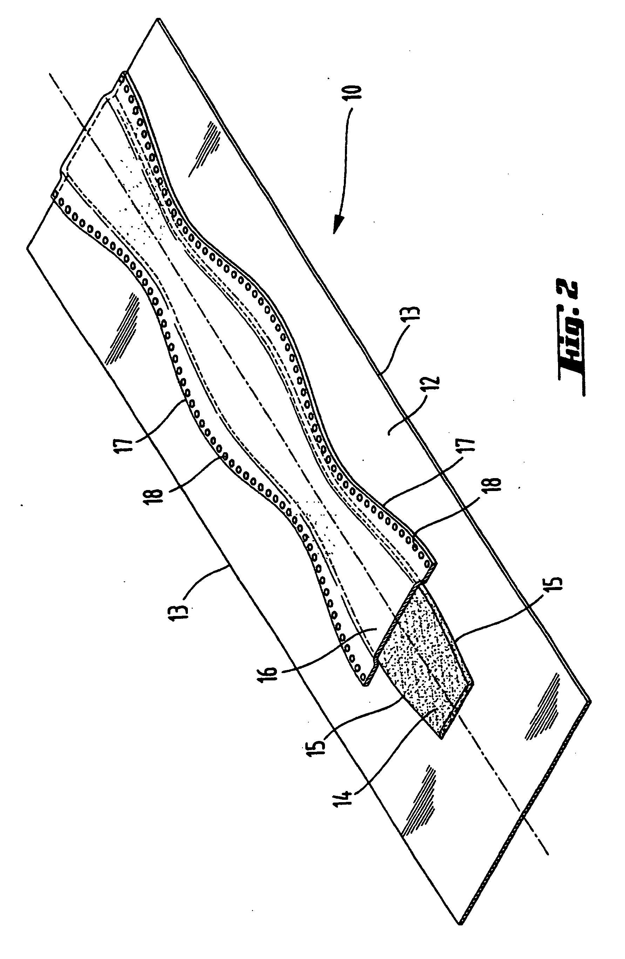

The present invention relates to a process for making a laminate which laminate comprises particulate material and which laminate is typically useful in disposable absorbent articles. The process steps required by the present invention will now be explained without limitation by reference to the embodiment of the process shown in figure 1 and the laminate resulting from a process according to the present invention shown in figure 2.

In figure 1 a film material (12) is provided at an unwind station (1). The film (12) is then transported downstream in a process direction. In a particle hopper (3) particulate material (14) is provided by continuously depositing the particulate material (14) on the film (12). This is typically done symmetrical with respect to the centre line of the film (12) and of the layer of particulate material (14).

As the next process step a continuos liquid pervious web (16) is provided at unwind stand (2) and introduced onto the particulate material layer (14) and also preferably centred along the symmetry axis in longitudinal, i. e. in process direction. The liquid pervious web and the liquid impervious film are combined at thermal bonding station (4) to form the laminate (10) according to the present invention. The laminate (10) can then be routed into a larger process to make disposable absorbent articles in accordance with a particular embodiment of the present invention or alternatively it can be wound up into rolls in order to store the laminate (10) until further use.

In figure 2 an embodiment of the laminate resulting from the process according to the present invention is shown. The laminate (10) comprises the film material (12) shown to have longitudinal side edges (13) and be wider than the liquid pervious substrate (16) and the particulate material layer (14). The particulate material layer (14) is not necessaryly continuos in longitudinal or process direction. A desired distance between individual patches of particulate material may be free of particulate material by intermittend deposition. The layer of particulate material is considered to extend between its longitudinal side edges (15). All particles are required to be deposited within the width Wp defined by the longitudinal side edges (15).

The longitudinal side edges (13) of the film and the longitudinal side edges (17) of the liquid pervious substrate (16) are spaced such that they overlap outside the width Wp between the longitudinal side edges (15) of the particulate material layer. In this region outside the longitudinal side edges (15) of the particulate material layer the film (12) and the liquid pervious substrate (16) are combined by thermal bonding which in this case is shown to be provided by spot welding (18).

As shown in figure 2 all materials of the laminate and therefore also the process according to present invention have an aligned centre line which is at the same time symmetry axis for the laminate and each individual material. The film material (12) has linear longitudinal side edges (13) while the liquid pervious substrate (16) has longitudinal side edges (17) which have a variable distance in transverse direction. Also the particulate material layer (14) has longitudinal edges (15) which are non-linear but experience a variable width which is following the maximum and minimum width in transverse direction of the liquid pervious substrate layer (16).

The thermal bonding provided at the thermal bonding station (4) is outside the width Wp of the particulate material layer (14) and follows the longitudinal side edge pattern of the liquid pervious substrate layer (16). The thermal bonding can be a provided as shown by spot welding but also could be provided by a continuous line or multitude of continuous lines and/or spots along the longitudinal side edges (17) of the liquid pervious substrate (16) or by any other typical and usual pattern used in the art of making disposable absorbent articles. The thermal bonding can be provided by high pressure welding, by ultrasonic means, by combination of heat and pressure or any other means typically used in the art of thermal bonding.

The equipment and process settings according to the present invention have of course to be adjusted and selected in order to the materials usual for the laminates according to the present invention. Therefore a brief description of the usual materials used in the field of disposable absorbent articles for both the disposable absorbent article as such and for the laminate in particular is provided in the following. The selection of process settings and equipment as well as alterations to process steps is then easily possible for the man skilled in the art and will at most require simple experimentation in order to result in a useful, energy conservative, robust and reliable, and economic process to provide laminates or disposable absorbent articles according to the present invention.

The disposable absorbent article is described below by reference to a sanitary napkin or catamenial, however baby diapers or adult incontinence articles are also included under the term disposable absorbent articles. The term "sanitary napkin", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. The disposable absorbent article is substantially flat prior to use.

Sanitary napkins with longitudinal side cuffs, which may be optionally elasticated, and sanitary napkins with a moderate curvature can also benefit from the present invention. Shaped absorbent cores to better conform to the width of the pudendal region, i.e. having a reduced width versus the front and/or rear end of the core, are generally desirable.

The terms "joined" or "affixed", as used herein, encompasses configurations whereby a first member is directly connected to a second member and configurations whereby a first member is indirectly connected to a second member by connecting the first member to intermediate members which in turn are connected to the second member.

A sanitary napkin preferably comprises a liquid pervious topsheet laminate, a liquid impervious backsheet laminate joined to the topsheet laminate, and an absorbent core laminate intermediate the topsheet laminate and the backsheet laminate. The sanitary napkin has two main surfaces, a body contacting or facing surface, and a garment facing or contacting surface. In order to indicate the general construction of sanitary napkins the 3 primary components are indicated to be laminates. However, the topsheet laminate, the backsheet laminate, and the core laminate may each be provided by a single structure.

The topsheet laminate is compliant, soft feeling, and non-irritating to the wearer's skin. Further, the topsheet laminate is liquid pervious, permitting liquid to readily penetrate through its thickness. A suitable topsheet laminate may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibres), synthetic fibres (e.g., polymeric fibres such as polyester, polypropylene, or polyethylene fibres); or from a combination of natural and synthetic fibres.

A preferred topsheet laminate is a formed film marketed on sanitary napkins by The Procter & Gamble Company of Cincinnati, Ohio as "DRI-WEAVE". In a preferred embodiment of the present invention, the body or exposed surface of the formed film topsheet laminate is hydrophilic so as to help liquid transfer through the topsheet laminate faster than if the body surface were not hydrophilic so as to diminish the likelihood that fluid will flow off the topsheet laminate rather than flowing into and being absorbed by the absorbent core laminate.

The liquid pervious substrate used in the process according to the present invention can be similar or even identical to the materials useful for the topsheet laminate described above. Preferably the liquid pervious substrate is a non-woven material. It may be absorbent, i. e. hydrophilic, or hydrophobic or made of hydrophobic fibres which have been rendered hydrophobic. The substrate can be thermally bonded non-woven or adhesively bonded. It also may be spunbonded, carded, or meltblown. The fibres can be those useful for the topsheet laminate and in the absorbent core laminate such as cellulosic fibres, tissues a. s. o. as will be explained below.

The backsheet laminate is substantially impervious to liquids (e.g., menses and/or urine) but otherwise similar to the topsheet laminate. The function of the backsheet laminate is to prevent exudates which may be expelled from or which inadvertently bypass the absorbent core laminate from contacting and soiling the user's undergarments. The backsheet laminate can thus comprise a woven or nonwoven material, polymeric films such as thermoplastic films of polyethylene or polypropylene, or composite materials such as a film-coated nonwoven material. Preferably, the backsheet laminate is a polyethylene film which is embossed and/or has a matte finished to provide a more clothlike appearance.

Preferably, the backsheet laminate also provides breathability to the absorbent article by being at least water vapour permeable, preferably air permeable, however, without compromising the main function of the backsheet laminate. The backsheet laminate can be a laminate material e.g. of a microporous film and apertured formed film or a single layer such as a microporous film alone. Breathability if desired can be limited to the periphery of the backsheet laminate or it can be across the whole backsheet laminate.

The backsheet laminate can be provided at least partially by the liquid impervious film used in the process of the present invention. In a preferred embodiment of the process according to the present invention the whole backsheet laminate is provided by the liquid impervious film (12). Therefore the liquid impervious film (12) can be selected from those materials useful for providing the inner most layer of the backsheet laminate.

In a preferred embodiment of the present invention the sanitary napkin is also provided with a panty fastening means. Typically, at least a portion of the outer surface of the backsheet laminate is coated with adhesive to form a panty fastening adhesive. The panty fastening adhesive is typically covered with a removable release paper or film in order to prevent the adhesive from drying out or adhering to another surface other than the panty prior to use.

In a preferred embodiment the absorbent core laminate comprises an absorbent element joined together and in face to face relationship with the liquid pervious substrate (16) of the present invention, this substrate (16) being positioned between the absorbent element and the backsheet laminate.

The absorbent element can be provided by a fibrous substrate of single or multi layer type as is usual in the art. The fibres can be hydrophilic or hydrophobic or mixed but have to provide absorbency to the absorbent core laminate. The absorbent element can comprise unbonded fibrous structures such as wood pulp or paper fluff or bonded fibrous structures such as tissues or airlayed webs or wetlayed webs. The bonding can be provided by polymeric meltable particles such as polyethylene particles or polypropylene particles. It can also be provided by latex coating or even by mechanical fibre entanglement or combinations thereof.

The absorbent element and the liquid pervious substrate (16) may be associated in any suitable manner to form the absorbent core laminate. Suitable manners include, but are not limited to, associating the absorbent element and the liquid pervious substrate with adhesives such as by spray-gluing or by applying lines or spots of adhesive between them. Alternatively, or additionally, the association between the layers may be achieved by fibre entanglement or by a plurality of discrete fusion bonds.

Alternatively, the liquid pervious substrate (16) together with the particulate material (14) may constitute the entire absorbent core laminate.

The absorbent capacity of the absorbent core laminate should be compatible with the intended body fluid loading for the absorbent article including the capacity provided by the liquid pervious substrate (16) and the particulate material (14).

Optionally, a sanitary napkin may have flaps which are adjacent to and extend laterally from the side edge of the absorbent core laminate. The flaps are configured to drape over the edges of the wearer's panties in the crotch region so that the flaps are disposed between the edges of the wearer's panties and the wearer's thighs. The flaps help serve to prevent soiling of the wearer's body and panties to keep the sanitary napkin properly positioned in the panty.

The flaps may be constructed of various materials and according to various designs all well known in the art. Preferred flaps that are suitable or adaptable to the sanitary napkin of the present invention are disclosed in U.S. Pat. No. 4,687,478 issued to Van Tilburg on Aug. 18, 1987 and U.S. Pat. No. 4,589,876 issued to Van Tilburg on May 20, 1986. Alternatively, a sanitary napkin may comprise components that naturally wrap the sides of a wearer's panties.

Characteristics and features usual for diapers or incontinence articles are well known in the art of absorbent articles and will be included in such articles. This includes without limitation elastics, closure mechanisms, leg barriers and others which are known in the art.

The particulate material component which is included in the absorbent articles of the present invention comprises preferably an odour control means; any suitable odour control means can be incorporated in the article of the present invention in any desired form such as granuals, capsules/microcapsules, or powder which are made according to techniques well known in the art. The preferred odour control materials are zeolites, carbon black and pH-buffers such as polyacrylates or borates.

## Claims

1. A process for making a laminate, said laminate comprising a particulate material and having continuous longitudinal edges, said process comprises the following steps:
- providing a continuous web of liquid impervious film, said film having a width Wf and longitudinal side edges;
- continuously depositing on said film a layer of particulate material having a width Wp such that a strip of said film comprises said particulate material, said layer having longitudinal side edges, wherein said width Wp of said layer of particulate material comprises all particles;
- providing a continuous web of liquid pervious substrate, said substrate having a width WI and longitudinal side edges;
- depositing said substrate onto said film such that it covers said particulate Material;
- continuous thermal bonding of said liquid pervious substrate and said film in a region of said film and of said liquid pervious web, said region being adjacent said longitudinal side edge of said layer of particulate material;
wherein said continuous web of liquid impervious film has linear longitudinal side edges and said layer of particulate material has non-linear longitudinal side edges and wherein said thermal bonding follows said non-linear longitudinal side edge contour.

2. A process according to claim 1 wherein both said continuous webs and said layer of particulate material each have a longitudinal centerline, both said continuous webs and said layer of particulate material being substantially symmetrical with respect to their respective longitudinal centerline and said longitudinal centerlines of both said continuous webs and of said layer of particulate material being aligned with each other to form a single centerline of said laminate.

3. A process according to any of the preceding claims wherein said particulate material comprises odour control material, preferably selected from zeolite, carbon black and a pH-buffer material.

4. A process according to any of the preceding claims wherein said liquid pervious substrate is fibrous, said fibrous substrate comprises fibers which are capable of thermobonding to said liquid impervious film.

5. A process according to any of the preceding claims wherein said continuous thermal bonding is provided by a pattern of spot welding cites.

6. A process according to any of the preceding claims wherein said width Wf is larger than said width WI.

7. A process according to claim 6 as part of a process for making absorbent sanitary articles said articles comprising a backsheet laminate, a liquid pervious topsheet laminate to be worn adjacent the wearer's skin and an absorbent core laminate deposited between said backsheet laminate and said topsheet laminate, said backsheet laminate and said core laminate being provided at least partially by said particulate material comprising laminate made with the process of claim 6 with said layer of particulate material layer and said web of liquid pervious substrate forming part of said core laminate.

8. A process according to claim 7 wherein said backsheet laminate consists of said liquid impervious film.

## Patentansprüche

1. Verfahren zur Herstellung eines Laminats, wobei das Laminat ein teilchenförmiges Material umfasst und kontinuierliche Längsränder aufweist, wobei das Verfahren die folgenden Schritte umfasst:
- Bereitstellen einer kontinuierlichen Bahn aus flüssigkeitsundurchlässiger Folie, wobei die Folie eine Breite Wf und Längsseitenränder aufweist;
- kontinuierliches Ablagern einer Schicht aus teilchenförmigem Material mit einer Breite Wp auf der Folie, sodass ein Streifen der Folie das teilchenförmige Material aufweist, wobei die Schicht Längsseitenränder aufweist, wobei die Breite Wp der Schicht aus teilchenförmigen Material alle Teilchen umfasst;
- Bereitstellen einer kontinuierlichen Bahn aus flüssigkeitsdurchlässigem Substrat, wobei das Substrat eine Breite Wl und Längsseitenränder aufweist;
- Anordnen des Substrats auf der Folie, sodass es das teilchenförmige Material bedeckt;
- kontinuierliches thermisches Verbinden des flüssigkeitsdurchlässigen Substrats und der Folie in einer Region der Folie und der flüssigkeitsdurchlässigen Bahn, wobei die Region benachbart zu dem Längsseitenrand der Schicht aus teilchenförmigem Material ist,
wobei die Bahn aus flüssigkeitsdurchlässiger Folie lineare Längsseitenränder aufweist und die Bahn aus teilchenförmigem Material nicht-lineare Längsseitenränder aufweist, und wobei das thermische Verbinden der nichtlinearen Längsseitenränderkontur folgt.

2. Verfahren nach Anspruch 1, wobei sowohl die kontinuierlichen Bahnen als auch die Schicht aus teilchenförmigem Material jeweils eine Längsmittellinie aufweisen, wobei sowohl die kontinuierlichen Bahnen als auch die Schicht aus teilchenförmigem Material im Wesentlichen symmetrisch in Bezug auf ihre jeweilige Längsmittellinie sind und die Längsmittellinien sowohl der kontinuierlichen Bahnen als auch der Schicht aus teilchenförmigem Material nach einander ausgerichtet sind, um eine einzige Mittellinie des Laminats zu bilden.

3. Verfahren nach einem der vorangehenden Ansprüche, wobei das teilchenförmige Material ein geruchsregelndes Material umfasst, das vorzugsweise ausgewählt ist aus Zeolith, Ruß und einem pH-Puffermaterial.

4. Verfahren nach einem der vorangehenden Ansprüche, wobei das flüssigkeitsdurchlässige Substrat faserig ist, wobei das faserige Substrat Fasern umfasst, die in der Lage sind, sich thermisch an die flüssigkeitsundurchlässige Folie zu binden.

5. Verfahren nach einem der vorangehenden Ansprüche, wobei die kontinuierliche thermische Verbindung durch ein Muster aus Punktschweißstellen geliefert wird.

6. Verfahren nach einem der vorangehenden Ansprüche, wobei die Breite Wf größer ist als die Breite Wl.

7. Verfahren nach Anspruch 6 als Teil eines Verfahrens zur Herstellung von absorbierenden Hygieneartikeln, wobei die Artikel umfassen ein Unterschichtlaminat, ein flüssigkeitsdurchlässiges Oberschichtlaminat, das angrenzend an die Haut des Trägers zu tragen ist, und ein Absorptionskernlaminat, das zwischen dem Unterschichtlaminat und dem Oberschichtlaminat angeordnet ist, wobei das Unterschichtlaminat und das Kernlaminat zumindest teilweise durch das teilchenförmige Material umfassende, nach dem Verfahren hergestellte Laminat gemäß Anspruch 6 gebildet sind, wobei die Schicht aus teilchenförmigem Material und die Bahn aus flüssigkeitsdurchlässigem Substrat einen Teil des Kernlaminats bilden.

8. Verfahren nach Anspruch 7, wobei das Unterschichtlaminat aus der flüssigkeitsundurchlässigen Folie besteht.

## Revendications

1. Procédé de fabrication d'un stratifié, ledit stratifié comprenant un matériau particulaire et ayant des bords longitudinaux continus, ledit procédé comprenant les étapes suivantes :
- fournir un voile continu de film imperméable aux liquides, ledit film ayant une largeur Wf et des bords latéraux longitudinaux ;
- déposer en continu sur ledit film une couche de matériau particulaire ayant une largeur Wp de telle sorte qu'une bande dudit film comprend ledit matériau particulaire, ladite couche ayant des bords latéraux longitudinaux, ladite largeur Wp de ladite couche de matériau particulaire comprenant toutes les particules ;
- fournir un voile continu de substrat perméable aux liquides, ledit substrat ayant une largeur WI et des bords latéraux longitudinaux ;
- déposer ledit substrat sur ledit film de telle sorte qu'il recouvre ledit matériau particulaire ;
- lier thermiquement en continu ledit substrat perméable aux liquides et ledit film dans une région dudit film et dudit voile perméable aux liquides, ladite région étant adjacente audit bord latéral longitudinal de ladite couche de matériau particulaire,
dans lequel ledit voile continu de film imperméable aux liquides a des bords latéraux longitudinaux linéaires et ladite couche de matériau particulaire a des bords latéraux longitudinaux non linéaires et dans lequel ladite liaison thermique suit le contour desdits bords latéraux longitudinaux non linéaires.

2. Procédé selon la revendication 1, dans lequel tant lesdits voiles continus que ladite couche de matériau particulaire ont chacun une ligne médiane longitudinale, lesdits voiles continus et ladite couche de matériau particulaire étant essentiellement symétriques par rapport à leur ligne médiane longitudinale respective et lesdites lignes médianes longitudinales desdits voiles continus et de ladite couche de matériau particulaire étant alignées les unes avec les autres de façon à former une seule ligne médiane dudit stratifié.

3. Procédé selon l'une quelconque des revendications précédentes, dans lequel ledit matériau particulaire comprend un matériau de contrôle des odeurs, de préférence choisi parmi une zéolite, du noir de carbone et un matériau de régulation du pH.

4. Procédé selon l'une quelconque des revendications précédentes, dans lequel ledit substrat perméable aux liquides est fibreux, ledit substrat fibreux comprenant des fibres qui sont capables sous l'effet de la chaleur de se lier audit film imperméable aux liquides.

5. Procédé selon l'une quelconque des revendications précédentes, dans lequel ladite liaison thermique continue est réalisée suivant un schéma de zones de soudage par point.

6. Procédé selon l'une quelconque des revendications précédentes, dans lequel ladite largeur Wf est plus grande que ladite largeur WI.

7. Procédé selon la revendication 6, en tant que composante d'un procédé de fabrication d'articles hygiéniques absorbants, lesdits articles comprenant un stratifié de feuille de fond, un stratifié de feuille de dessus perméable aux liquides destiné à être porté sur un plan adjacent à la peau de l'utilisateur, et un stratifié d'âme absorbante déposé entre ledit stratifié de feuille de fond et ledit stratifié de feuille de dessus, ledit stratifié de feuille de fond et ledit stratifié d'âme étant constitués au moins partiellement dudit stratifié comprenant un matériau particulaire fabriqué à l'aide du procédé selon la revendication 6, ladite couche de matériau particulaire et ledit voile de substrat perméable aux liquides formant une partie dudit stratifié d'âme.

8. Procédé selon la revendication 7, dans lequel ledit stratifié de feuille de fond est constitué dudit film imperméable aux liquides.
